(19) Europäisches Patentamt
European Patent Office
Office européen des brevets

(11) **EP 3 246 888 A1**

(12) **EUROPEAN PATENT APPLICATION**
published in accordance with Art. 153(4) EPC

(43) Date of publication:
**22.11.2017 Bulletin 2017/47**

(51) Int Cl.:
***G08B 21/04*** (2006.01)

(21) Application number: **16847840.2**

(22) Date of filing: **03.06.2016**

(86) International application number:
**PCT/CN2016/084730**

(87) International publication number:
**WO 2017/049958 (30.03.2017 Gazette 2017/13)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(30) Priority: **25.09.2015 CN 201510629054**

(71) Applicant: **Guangdong Appscomm Co., Ltd.
Guangzhou, Guangdong 510663 (CN)**

(72) Inventor: **QIAO, Lijun
Guangzhou
Guangdong 510663 (CN)**

(74) Representative: **Sebastian, Jens
ABACUS
Patentanwälte
Lise-Meitner-Straße 21
72202 Nagold (DE)**

(54) **PARALYSIS DETECTION AND ALARMING APPARATUS AND PROCESSING METHOD THEREOF**

(57) The present invention discloses a device for monitoring and preventing slump and a processing method thereof. The device includes: an alarm module, a signal collection module, and a signal processing module, wherein the signal collection module is used for collecting human-body posture behavior data and biometrics data; the signal processing module includes a slump detection unit configured to perform analysis processing according to the human-body posture behavior data and biometrics data to judge a state of the human body, and output a help signal to the alarm module when judging that a slump occurs to the human body; and the alarm module generates and outputs help message according to the help signal. The device and method according to the invention, perform slump detection through the human-body posture behavior data and biometrics data, thus the behavior characteristics of the human body are considered effectively, and higher accuracy is achieved.

EP 3 246 888 A1

Opening and initializing a device — S111

Setting basic information of a wearer through a terminal device — S112

A data collection module collects device wearing state information data, and instructs a user by voice — S113

Whether the device is worn correctly — S114 — No / Yes

The data collection module collects data in real time, analyzes and determines whether a human body has a slump risk and whether a slump occurs to the human body — S115

Whether the slump risk exists — No / Yes

Informing the slump risk to the user through a man-machine interaction module

Whether a slump occurs — S116 — No / Yes

Sending a help signal to the alarm module, and performing alarm processing through the alarm module — S117

Fig. 11

**Description**

**TECHNICAL FIELD**

The present invention relates to an intelligent security product, and more particularly, to a device for monitoring and preventing slump and a processing method thereof.

**BACKGROUND**

With the change in the pace of life, the lonely elderly and the left-behind elderly have become a large unneglectable group. More young people cannot accompany with the elderly all the time due to working and other reasons, which causes that the elderly cannot be rescued in time in case of slumping and this deteriorates the illness. Therefore, it is very necessary to provide an intelligent device that can be worn everywhere and is capable of monitoring a physical condition of the elderly in real time for the elderly, so that the elderly can get help within the shortest time.

There are a plurality of monitoring devices currently, all of which are implemented using a technical means of tumble monitoring. While a common way to achieve tumble monitoring is to use an acceleration sensor to detect an acutely changed signal, which appears at the moment when a body hitting on the ground during tumbling, and a relatively static signal, which appears in a period when the body cannot move after tumbling, to recognize whether a slump occurs. However, the slump indicates a situation that limbs are out of control of subjective consciousness because of some shock symptoms, in more cases, the slump is caused by the body itself, and the posture feature of which is not always changed into lying flat from standing (during the change, the acutely changed signal appears firstly, and then comes the relatively static signal), but behaves in various forms, for example in the form that slumping when one is in a sitting posture and keeping the sitting posture when slumped, or in the form that slumping against a wall and then sitting on the ground slowly, etc. Under such kind of situation, the slump cannot be detected by means of tumble monitoring. Moreover, a misinformation problem caused by the device departing from the body artificially or accidentally is not solved by the current monitoring device.

**SUMMARY**

According to one aspect of the present invention, a device for monitoring and preventing slump is provided, which comprises an alarm module, a signal collection module, and a signal processing module. The signal collection module is used for collecting human-body posture behavior data and human-body biometrics data. The signal processing module comprises a slump detection unit configured to perform analysis processing according to the collected human-body posture behavior data and human-body biometrics data to determine a state of the human body, and output a signal for rescue to the alarm module when determining that a slump occurs to the human body. The alarm module generates a help message according to the signal and outputs same.

The monitoring device according to the invention performs slump detection by means of human-body posture behavior data and human-body biometric data. In this way, the behavior characteristics of the human body are effectively considered, thus higher accuracy is achieved.

In some embodiments, the signal processing module further comprises a wearing state detection unit, the signal collection module is further used for collecting wearing state information of the device, and the wearing state detection unit is configured to perform analysis processing according to the wearing state information of the device and output a wearing state flag. The slump detection unit performs human body slump detection and outputs the signal to the alarm module according to the outputted wearing state flag. Therefore, whether a wearing direction and/or wearing position of the device is correct or not may be detected, so as to avoid the badness of misinformation caused by incorrect wearing, and further improve the accuracy of the slump detection.

In some embodiments, the signal collection module is also used for collecting environmental information from a vicinity of the human body, and the signal processing unit further includes a slump prevention unit configured to judge the state of the human body according to the human-body posture behavior data, the human-body biometrics data and the environmental information, calculate a slump risk grade in a weighted manner according to the judgment, and output the slump risk grade. In this way, the device can make further improvement on monitoring the state of the human body, and a user can be prompted in advance in case of any slump risk, so that slump may be prevented and the monitoring security of the user can be improved.

In some embodiments, the device further includes a positioning module and a wireless communication module, the positioning module collects location information of the device, and the alarm module sends a help message containing the location information to a remote terminal through the wireless communication module. Therefore, a monitor may be informed in time, meanwhile the location information of a wearer is provided, thus it facilitates the user to get effective help in time.

In some embodiments, the signal collection module collects information through sensors, including a sensor used for collecting human-body the posture behavior data, a sensor used for collecting the human-body biometric data, a sensor used for collecting the wearing state information of the device, and a sensor used for collecting the environmental information from a vicinity of human body, wherein the sensor used for collecting the human-body posture behavior data comprises a tri-axis acceleration sensor or comprises a tri-axis acceleration sensor, an air pressure sensor, and a gyroscope; the sensor used for collecting the human-body biometric data comprises one of or a combination of more than two of a heart rate sensor, a photoelectric blood pressure sensor, a temperature sensor, a humidity sensor, and an oxygen saturation sensor; the sensor used for collecting the wearing state information of the device comprises one of or a combination of more than two of a temperature sensor, a bioelectricity sensor, a pressure sensor, and a tri-axis acceleration sensor; and the sensor used for collecting the environmental information from a vicinity of human body comprises one of or a combination of more than two of an air pressure sensor, a temperature sensor, a humidity sensor, and an air velocity sensor. Thus, corresponding data may be collected so as to perform monitoring analysis.

In some embodiments, a man-machine interaction module is also provided, which includes one of or a combination of more than two of a touch screen, a button, and a voice player. The slump prevention unit outputs the slump risk grade through the man-machine interaction module. Therefore, the user can input user information through the touch screen to facilitate the device to perform analysis processing according to the user information, and also can ask for help or cancel the misinformation for help through a button when the device spreads misinformation because of wrong detection. It can effectively improve the user experience, and is quick and convenient.

According to one aspect of the present invention, an processing method of a device for monitoring and preventing slump is also provided, wherein the device comprises a positioning module, a wireless communication module, an alarm module, a signal collection module, and a signal processing module, and the processing method includes:

collecting, by the signal collection module, human-body posture behavior data and human-body biometrics data in real time, and outputting same to the signal processing module;
performing, by the signal processing module, analysis processing according to the human-body posture behavior data and human-body biometrics data, judging a state of the human body, and outputting a signal for help to the alarm module when judging that a slump occurs to the human body; and
sending, by the alarm module, a help message containing location information to a remote terminal according to the signal.

According to the method of the present invention, slump detection is performed by means of the human-body posture behavior data and human-body biometrics data. Thus behavior characteristics of the user can be considered to effectively detect whether a slump occurs to the human body, and higher accuracy can also be achieved.

In some embodiments, the processing method further includes:

collecting, by the signal collection module, wearing state information of the device in real time, and outputting same to the signal processing module;
performing, by the signal processing module, an analysis according to the wearing state information, judging a wearing state of the device, and outputting a wearing state flag according to the wearing state of the device; and
reading, by the signal processing module, the wearing state flag to determine, and when determining that the wearing state flag indicates the device is worn correctly, performing a judgment of human body slump state and outputting the signal for help to the alarm module according to the slump state. Whether the human body wears the device may be judged through temperature, and whether the position and direction of the device worn by the human body are correct may be judged through pressure and acceleration, thus detection on whether the device is worn or is worn correctly is achieved, and the badness of misinformation caused by the device is also avoided. Moreover, the collection of user behavior information and human body slump detection are only performed when the device is worn correctly, which makes it possible to reduce unnecessary data processing operation and improve efficiency.

In some embodiments, the signal collection module collects the human-body posture behavior data through a tri-axis acceleration sensor, and collects the human-body biometrics data through one of or a combination of more than two of a heart rate sensor, a photoelectric blood pressure sensor, a temperature sensor, a humidity sensor, and an oxygen saturation sensor. In this case, performing analysis processing according to the human-body posture behavior data and human-body biometrics data, and judging the state of the human body through the signal processing module are implemented by :

reading tri-axis acceleration data collected by the tri-axis acceleration sensor, and calculating a sum, which is referred to as ACC_SUM, of changes of the tri-axis acceleration;
judging whether the sum ACC_SUM of changes of the tri-axis acceleration is smaller than a given threshold, and setting a body posture flag as TRUE if the sum ACC_SUM is smaller than the given threshold; and
reading the human-body biometrics data, and judging whether the human-body biometrics data is greater than the upper threshold set in advance or is smaller than the lower threshold set in advance, determining the severity level of slump of the human body according to the default level of a threshold and outputting a human body slump flag when judging that the human-body biometrics data is greater than the upper threshold or smaller than the lower threshold. Therefore, whether the body of the user enters a silent period and whether the human body characteristic is abnormal may be detected, so that the slump state of a human body is determined.

In some embodiments, the method further includes: collecting, by the signal collection module, environmental information from a vicinity of the human body in real time, and outputting same to the signal processing module; and performing, by the signal processing module, analysis processing according to the human-body posture behavior data, human-body biometrics data and environmental information, judging the state of the human body, calculating the data in a weighted manner according to the judgment result, and outputting a slump risk grade. In this way, it is able to prevent the slump by means of detecting the risk of slump, so that negative effects caused by slump and the occurrence of slump are reduced.

In some embodiments, the signal collection module collects the human-body posture behavior data through the tri-axis sensor, gyroscope and air pressure sensor, collects the human-body biometrics data through one of or a combination of more than two of the heart rate sensor, photoelectric blood pressure sensor, temperature sensor, humidity sensor, and oxygen saturation sensor, and collects the environmental information from a vicinity of the human body through one of or a combination of more than two of the air pressure sensor, temperature sensor, humidity sensor and air velocity sensor. In this case, the signal processing module performing analysis processing according to the human-body posture behavior data, human-body biometrics data and environmental information, judging the state of the human body, calculating the data in a weighted manner according to the judgment result, and outputting the slump risk grade can be implemented by:

reading the human-body posture behavior data, human-body biometrics data and environmental information from a vicinity of human body, judging whether the datum are abnormal respectively, and setting the risk flag of slump as TRUE when one of the datum is abnormal; and
reading a value of a flag representing the alarm state of slump, which is referred to as SLUMP_WARN_FLAG, to judge, obtaining the human-body biometrics data when the value of SLUMP_WARN_FLAG is TURE, and calculating the current risk grade of slump according to a formula

$$score = \sum_{i=0}^{n} a_i * \left| D_i - DH_i \right|$$

or

$$score = (w_0 * \max(1, \frac{age}{age_0}) + w_1 * \frac{weight}{height * weight}) * \sum_{i=0}^{n} a_i * \left| D_i - DH_i \right| \ ;$$

wherein $a_i$ is a weighted value of $i^{th}$ biometrics data, $D_i$ is a value of $i^{th}$ biometrics data collected, $DH_i$ is a value of $i^{th}$ biometrics data under a normal condition, n is the number of a sensor configured to collect the biometrics data in the signal collection module, age is age, height is height, weight is weight, $w_o$ and $w_1$ are weighted values, and $age_0$ is a given standard age value. Therefore, the slump risk may be quantified, and the risk grade may be judged and prompted correspondingly according to the quantification result.

## BREIF DESCRIPTION OF DRAWINGS

Fig. 1 is a schematic diagram illustrating an exterior structure of a device for monitoring and preventing slump, according to an embodiment of the present invention;
Fig. 2 is a schematic diagram illustrating a frame structure of modules in the device for monitoring and preventing slump, according to an embodiment of the present invention;
Fig. 3 is a flow chart of a method for recognizing and detecting slump, according to an embodiment of the present invention;

Fig. 4 is a flow chart of a method for detecting whether the device is worn correctly, according to an embodiment of the present invention;

Fig. 5 is a flow chart of a method for preventing slump, according to an embodiment of the present invention;

Fig. 6 is a working flow chart of the device for monitoring and preventing slump, according to an embodiment of the present invention;

Fig. 7 is a working flow chart of the device for monitoring and preventing slump, according to an embodiment of the present invention;

Fig. 8 is a working flow chart of the device for monitoring and preventing slump, according to another embodiment of the present invention;

Fig. 9 is a working flow chart of the device for monitoring and preventing slump, according to another embodiment of the present invention;

Fig. 10 is a working flow chart of the device for monitoring and preventing slump, according to another embodiment of the present invention; and

Fig. 11 is a working flow chart of the device for monitoring and preventing slump, according to another embodiment of the present invention.

## DETAILED DESCRIPTION

The present invention will be further described in details hereunder with reference to the drawings.

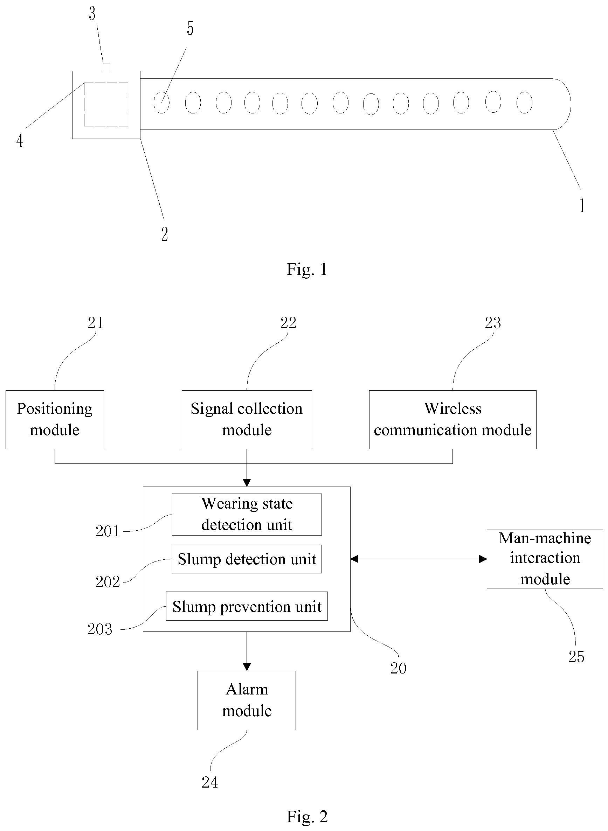

Fig. 1 schematically illustrates an exterior structure of a device for monitoring and preventing slump, according to one embodiment of the present invention. As shown in Fig. 1, the device includes a belt 1 and a belt buckle 2, one end of the belt 1 and belt buckle 2 are fixedly connected together, and the other end of the belt 1 and belt buckle 2 are fastened to each other when wearing it. The way to connect and fasten the belt 1 and belt buckle2 is the same as that of an ordinary waist belt. A button 3 is arranged on the belt buckle2, by pressing which a user can start a human-machine interaction. A pressure sensor 5 of the device is uniformly distributed along the belt 1, and all other function modules (such as a signal collection module, a signal processing module, a positioning module, or the like remained) are internally arranged on an integrated chip 4 in the belt buckle 2. In practical application, the user may implement daily monitoring of a wearer through wearing the waist belt as shown in Fig. 1. Since the waist belt is a necessity for daily dressing of most people, it is convenient to carry without any attachment feeling, is difficult to forget, and is very handy.

Fig. 2 schematically illustrates a frame structure of modules arranged in the belt of the device. As shown in Fig. 2, the device includes a signal processing module 20, a positioning module 21, a signal collection module 22, a wireless communication module 23, and an alarm module 24. The positioning module 21 is implemented by means of GPS, compass, mobile base station, or other location-based technology to provide geographical position information of the user. The wireless module 23 is a chip or a module that may be communicated with a mobile terminal device wirelessly, such as GSM communication unit, a bluetooth communication unit, etc., and is used for implementing data interaction with a remote terminal (such as a mobile phone, a computer, an IPad, and other terminals). The alarm module 24 is configured to send a help message to the remote terminal through the wireless communication module 23 when receiving a signal for help, so as to inform a guardian that a slump occurs to the user and the user needs help, wherein the help message includes the geographical position information obtained by the positioning module 21 and a specific content for help. The signal collection module 22 is configured to collect device wearing state information and user behavior information in real time, and provide the information to the signal processing module to perform human body slump detection analysis. The signal collection module 22 is mainly implemented by means of various sensors, including sensors configured to collect the wearing state information and sensors configured to collect the user information. The sensors configured to collect the wearing state information comprise a tri-axis acceleration sensor, a temperature sensor, a human body bioelectricity sensor, a pressure sensor, or the like, and the sensors configured to collect the user information include sensors configured to collect the human-body posture behavior data, sensors configured to collect the human-body biometrics data, and sensors configured to collect the environmental data from a vicinity of human body. The sensors configured to collect the human-body posture behavior data comprise a tri-axis acceleration sensor, a gyroscope, an air pressure altitude sensor, or the like; the sensors configured to collect the human-body biometrics data comprise a heart rate sensor, a photoelectric blood pressure sensor, an infrared temperature sensor, a humidity sensor, an oxygen saturation sensor, or the like; and the sensors configured to collect the environmental data from a vicinity of human body include an air pressure sensor, a temperature sensor, a humidity sensor, an air velocity sensor, or the like. The signal processing module 20 is a micro-processor like a MCU, etc., which comprising a slump detection unit 202. The slump detection unit 202 is used for performing slump detection according to the human-body posture behavior data and human-body biometrics data collected by the signal collection module 22, and outputting a signal for help to the alarm module 24 when judging that a slump occurs, so as to start the alarm module 24 to send the help message to the remote terminal device of the guardian through the wireless communication module 23.

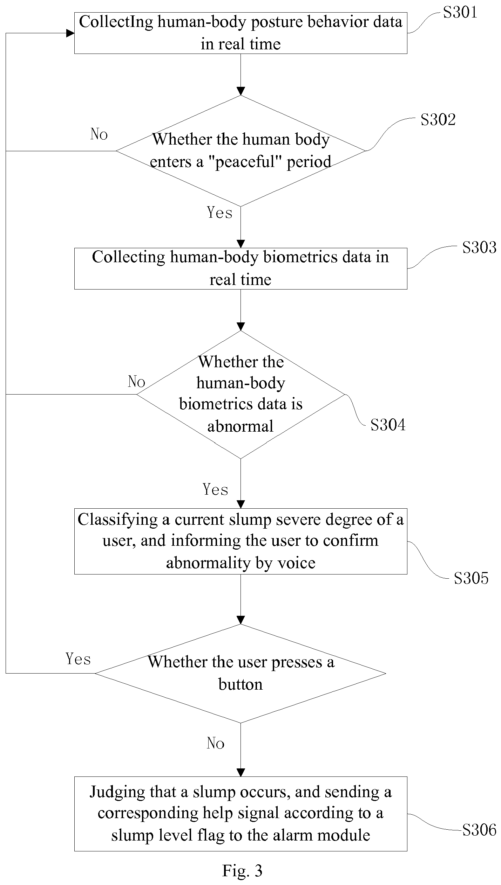

Fig. 3 schematically illustrates a method for recognizing and detecting slump performed by the slump detection unit 202 according to the human-body posture behavior data and human-body biometrics data collected by the signal collection module 22, according to one embodiment of the present invention. Taking the sensor configured to collect the human-body posture behavior data being a tri-axis acceleration sensor for example, as shown in Fig. 3, the method includes the following steps.

In step S301, the operation that collecting the human-body posture behavior data in real time is performed.

In the course of use, the signal collection module 22 collects the human-body posture behavior data in real time through the tri-axis acceleration sensor and saves the human-body posture behavior data (i.e., tri-axis acceleration data) collected in a period of time (such as 4s) through a form of FIFO (First In First Out);

In step S302, the operation that determining whether the human body enters a "peaceful" period is performed.

The slump detection unit 202 calculates a sum of changes of the tri-axis acceleration in the period of time, using formula $ACC\_SUM = \sum_{i=0}^{n-1} (\left|(AX_{i+1} - AX_i)\right| + \left|(AY_{i+1} - AY_i)\right| + \left|(AZ_{i+1} - AZ_i)\right|)$, according to the tri-axis acceleration data saved. In the formula, n is the number of the acceleration data collected in the period of time. An acceleration threshold TH4 is given, and whether the sum of the tri-axis acceleration changes ACC_SUM in the period of time is smaller than the given threshold TH4 is determined, i.e., whether a condition that ACC_SUM<TH4 is satisfied. For TH4 corresponds to the peaceful period after the occurrence of slump, and the acceleration change of the human body during this period is very small, thus it can be set as a relatively small value, for example approaching to 0.1g (g is an acceleration of gravity). If the condition is satisfied, it indicates that the body enters the peaceful period during a period of time, which is the usual status occurs after occurrence of normal slump. It can be determined that the human body has entered the peaceful period if the condition above is satisfied, thus the state flag of the human body, for example it can be referred to as BODY_FLAG, is set as TURE.

In step S303, the operation that collecting the human-body biometrics data in real time is performed.

The slump detection unit 202 reads the state flag of the human body to determine, and when determining that the state flag of the human body is TURE, the signal collection module 22 collects the human-body biometrics data in real time. The signal collection module 22 collects the human-body biometrics data through one of or a combination of more than two of a heart rate sensor, a photoelectric blood pressure sensor, a infrared temperature sensor, a humidity sensor, and an oxygen saturation sensor.

In step S304, the operation that determining whether the human-body biometrics data is abnormal is performed.

The slump detection unit 202 performs analysis according to the human-body biometrics data collected, determines whether the current body state of the user is abnormal. Usually, when a slump occurs to the elderly, the biometrics data is usually abnormal, for example, characteristics of "abnormal sweating", accelerated heart beat, increased blood pressure, or the like may be accompanied usually; and meanwhile, the body is in a "peaceful" state.

The slump detection unit 202 obtains the human-body biometrics data comprising heart beat, blood pressure, body temperature, humidity on body surface, oxygen saturation, or the like, according to the sensors above to determine. When determining that corresponding biometrics data is greater than the upper limit of thresholds set in advance or smaller than the lower limit of thresholds set in advance, it is concluded that a slump occurs, thus the slump state flag SLUMP_FLAG is set as TRUE. The given threshold may be set according to the basic information (such as age, sex, medical history, etc.) of a wearer set by the user in the combination of medical science. For the specific value of the biometrics data threshold is well known in the medical field, it will not be introduced in details herein.

In step S305, the operation that classifying the current slump severe degree of the user and informing to the user by voice to conduct abnormity confirmation is performed.

When the human-body biometrics data of the user is greater than the given threshold, i.e., when the slump state flag is read and judged to be TRUE, the device plays abnormity confirmation information by voice, such as playing a voice prompt "whether you feel uncomfortable, if not, please press the button", to wait the user for confirmation. If the user presses the button 3, then it indicates that the misjudgment occurs, and the slump state flag is set as FALSE. If the user does not press the button 3 during a period of time such as 5s, then it indicates that the slump really occurs to the human body, in this case, the slump detection unit 202 compares the corresponding biometrics data with a given slump degree index threshold in a descending order, and outputs the slump level flag which shows the corresponding severe degree. For example, the slump degree index threshold is set as follows according to the heart beat, humidity, and blood pressure: high degree (HUM_TH1/BLOODPRESSURE_TH1/HEART_RATE_TH1), middle degree (HUM_TH2/BLOODPRESSURE_TH2/HEART_RATE_TH2), and lower degree (HUM_TH3/BLOODPRESSURE_TH3/HEART_RATE_TH3). The slump detection unit 202 compares the corresponding biometrics data with an index threshold showing a high slump degree firstly, and then compares the data with index thresholds showing middle and lower slump degrees. When the biometrics data matches with an index threshold showing a some degree, the corresponding slump level flag is set as TRUE; for example, if the slump degree index threshold showing a high degree is matched, the slump level flag SLUMP_HIGH is set as TRUE, if the slump degree index threshold showing a middle degree is matched, the slump level flag SLUMP_MIDDLE is set as TRUE, and if the slump degree index threshold showing a lower degree is matched, the slump level flag SLUMP_LOW is set as TRUE.

In step S306, the operation that when determining that the slump occurs, sending a corresponding help signal to the alarm module according to the slump level flag is performed.

The slump detection unit 202 reads the slump level flag to judge, when the slump level flag of some degree is TRUE, the slump of corresponding degree is judged to occur, and the help signal of corresponding slump level is sent to the alarm module according to the slump level flag. For example, when SLUMP_LOW is TRUE, a character "L" is sent to the alarm module, and the alarm module sends the help message to the basic guardian according to the character "L"; and when SLUMP_HIGH is TRUE, a character "H" is sent to the alarm module, and the alarm module sends the help information (including geographic position information and specific help content) to all the guardians and the hospital through the wireless communication module according to the character "H", so that the wearer can get timely help in accordance with the slump degree, which may help the patient effectively.

It should be illustrated that during slump detection, the given biometrics threshold and the given slump degree index threshold may be set according to medical knowledge, or may be obtained by means of training a sample base set in the device. The sample base includes samples of common features of people and data sample of the user, and the threshold obtained through training the samples can provide parameters suitable for the self-owned characteristics of each user, which has higher accuracy. In the device provided with the sample base, when misjudgment happens in step S305, the corresponding biometrics data may be saved as a positive example in the sample base at the same time, which facilities to prevent the misjudgment to happen again, thus it can provide more accurate slump monitoring to the user. A method for training samples to obtain a suitable threshold may be implemented with reference to the prior art through statistics, which will not be elaborated herein.

The slump detection method provided by the embodiment needs to detect the human-body posture behavior data and biometrics data at the same time, it complies with the situations that a section of "acutely changed" signal led by hitting the ground by the body in slumping may not appear, and the final posture may not be a static condition of lying flat, and can comprehensively consider the behavior characteristic and biological sign characteristic data of the user. Thus the detection method according to the present invention can detect the human body slump state more accurately and effectively comparing with the tumble detection manner, and it facilitates the slump monitoring of the wearer, so that the wearer can send out a help request in the first place after slumping and get help. Meanwhile, the slump detection method according to the embodiment further classifies the slump severe degree based on the biometrics index thresholds of the human body, and different help contents are adopted according to the slump patients of different severe degrees, which is more convenient for the user, and improves the user experience.

Meanwhile, considering that the device wearing condition is further an important factor affecting the detection accuracy excepting the accuracy on algorithm aspect, the present invention provides the solution for misinformation caused under the condition that the device is not worn or is worn incorrectly meanwhile. As shown in Fig. 2, the signal processing module 20 further includes the wearing state detection unit 201. The wearing state detection unit 201 is configured to perform analysis processing according to the wearing state information of the device collected by the signal collection module 22, and output the wearing state flag. The slump detection unit 202 reads the wearing state flag to judge, when the wearing state flag is that the device is worn correctly (i.e., TURE), the user behavior data is collected for analysis and detection, and a help signal is outputted to the alarm module 24 when a slump occurs (refer to the method part of Fig. 3 in the text above for the specific methods of analysis, detection, and asking for help). In practical application, the signal collection module 22 collects the device wearing state information through the senor configured to collect the wearing information of the user, and the sensor configured to collect the wearing information may be one of or any combination of more than two of the tri-axis acceleration sensor, the temperature sensor, the human body bioelectricity sensor, the pressure sensor, etc. The wearing state detection unit 201 may detect whether the device is worn or is worn correctly only according to one of the pressure data, the tri-axis acceleration and temperature data above, or may select a combination of more than two of the datum at the same time to perform detection. When the combination of more than two of the datum are selected to conduct detection, as long as the detection result of any manner is to be worn incorrectly, the wearing state flag is set as FALSE. For example, whether the device is worn may be detected through the temperature data firstly, and when the temperature data cannot be detected (that is having the condition that the temperature difference between the human body temperature and the external temperature is close), whether the device is worn is then judged through the human body bioelectricity signal. If judging that the device is worn, then whether the wearing position is correct is judged by means of comparing the pressure data. If determining that the wearing position is correct, then whether the wearing direction is correct is judged according to the tri-axis acceleration. And if all the three conditions are correct, then the device is judged to be worn correctly, and the wearing state flag is set as TRUE. Otherwise, the wearing state flag is set as FALSE, and the data collection is conducted repeatedly. Performing data collection and detection through the manner of combining different sensors can effectively improve the accuracy of device wearing detection, and the more sensors are used, the more accurate the detection result will be.

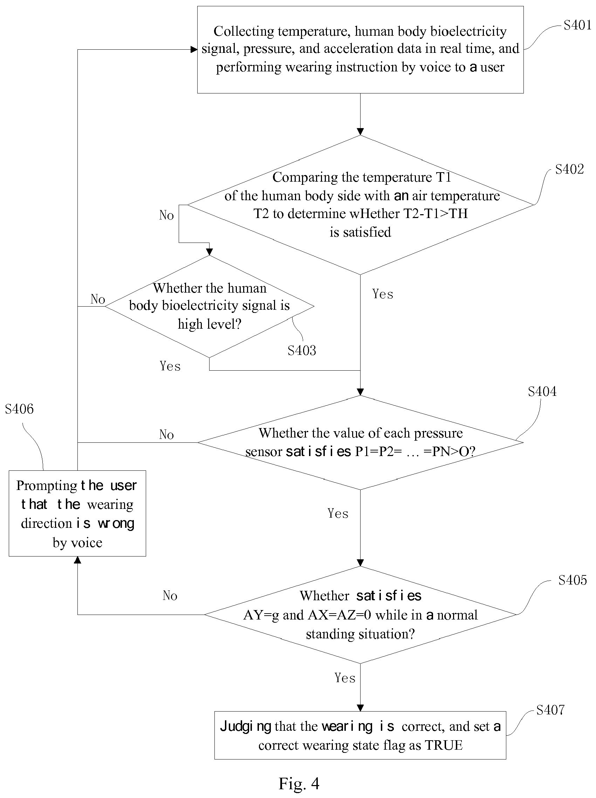

Fig. 4 schematically illustrates a method for detecting whether the detection device is worn correctly, according to one embodiment of the present invention. Taking the sensor used for detecting the wearing information including a tri-axis acceleration sensor, a temperature sensor, a human body sensor, and a pressure sensor at the same time for example, and as shown in Fig. 4, the method includes the follows steps.

In step S401, temperature, human body bioelectricity signal, pressure, and acceleration data are collected in real time, and a user is instructed to wear the device by voice.

The signal collection module collects tri-axis acceleration data AX, AY, and AZ in real time through the tri-axis acceleration sensor, collects pressure data P1, P2, ..., and PN along the circle of the wearing part in real time through the pressure sensor, collects temperature data T1 close to a human body side and temperature data T2 exposed to an air side in real time through the temperature sensor, and collects a human body bioelectricity signal in real time through the human body bioelectricity sensor. Meanwhile the wearing method instructing the user to wear the device is played by a voice module of the device.

It should be illustrated that the device according to the present invention is designed as a waist belt, thus to achieve the object of detecting the pressure on the wearing part of the device, the pressure sensor needs to be set as being uniformly distributed on the belt of the device along the circle of the wearing part in a matrix manner, with the number of N=L/D, wherein, L is length information of a circle of the wearing part of the user (obtained according to basic information inputted by the user), and D is a distance of the pressure sensors distributed in a matrix manner. However, in order to detect the air temperature and the human body temperature, two temperature sensors are set according to the embodiment. Since the temperature sensor itself has directional properties (facing towards the human body side and the outside), the direction of one temperature sensor is set to face towards the human body side, so as to be capable of collecting the human body temperature, and the direction of the other temperature sensor is set to face towards the air side, so as to be capable of collecting the environment temperature.

In step S402, the operation that comparing the human body side temperature T1 with the air side temperature T2 to determine whether T1-T2>TH is satisfied is performed.

If the device is not worn in the human body, then T1=T2 theoretically, but an error of about 0.5° exists actually, but if the device is worn in the human body, then one side is the air temperature, and another side is the human body temperature, this leads to a temperature difference between the two sides, thus according to which the object of detecting whether the device is worn correctly should be achieved. Based on the fact of the temperature difference, a threshold TH is given, and T1 and T2 are compared to detect whether the temperature difference is greater than the given threshold. If the temperature difference is greater than the given threshold, step S404 is conducted, and if not, step S403 is conducted.

In step S403, whether the human body bioelectricity signal is high level is judged.

It should be illustrated that, since the condition that the external temperature is close to the human body temperature exists, as a preferred embodiment, the human body bioelectricity sensor may be added in the signal collection module to conduct further detection, which specifically is that when the temperature difference between T1 and T2 is very small, the human body bioelectricity signal is collected to judge whether the human body bioelectricity signal is high level. If the human body bioelectricity sensor outputs the high level, it indicates that the human body wears the device, and the pressure detection of step S404 is then performed. Otherwise, the data collection of step S401 is conducted continuously. In practical application, the human body bioelectricity sensor may further used as an alternative solution of the temperature sensor to judge whether the device is worn, that is, the temperature sensor is replaced by the human body bioelectricity sensor, and the human body bioelectricity signal is judged instead of the temperature data. The specific combination manner can be that select any one of two sensors or combine the two sensors for use, it is not limited by the embodiment according to the present invention.

In step S404, whether the value of each pressure sensor is in accordance with P1=P2=...=PN>0 is judged.

If the human body wears the device correctly, the pressure values of a circle of the waist of human body should be equal and should be equal to pressure value P when the degree of tightness is proper, i.e., P1=P2=...=PN=P>0, thus whether the wearing part of the device is correct and whether the degree of tightness is proper may be judged through judging whether the condition is satisfied. If the condition is satisfied, step S405 is performed. Otherwise, the data collection of step S401 is conducted repeatedly.

In step S405, whether the tri-axis acceleration value is in accordance with AY=g and AX=AZ=0 when the human body is in a normal standing state is judged.

Under a normal condition, when the human body is upright, the correct wearing manner shall be that the acceleration value of only one axis is g (acceleration of gravity) and the acceleration value of other two axes shall be 0. It is provided that Y axis represents the body direction of the human body in standing state, that is AY=g and AX=AZ=0, and if the condition is satisfied, it indicates that the wearing direction of the device is correct, and then step S407 is conducted. Otherwise, step S406 is conducted.

In step S406, the user is prompted by voice that the wearing direction is incorrect.

A voice prompt is played to prompt the user that the wearing direction is incorrect, and the data collection of step S401 is conducted continuously.

In step S407, the device is judged to be worn correctly, and the correct wearing state flag is set as TRUE.

If the judgment conditions above are satisfied at the same time, then it indicates that the device has been worn, and both the wearing position and direction are correct. In this case, the correct wearing state flag WEAR_FLAG is set as TRUE, and then the device may perform the human-body state detection according to the wearing flag which indicates that the device is worn correctly.

The misinformation caused by the device that is not worn or is worn incorrectly may be avoided through detecting the device wearing condition, so as to improve the accuracy of slump detection and emergency alarm. Moreover, the state of the human-body state detection is performed only when the device is worn correctly, which can reduce unnecessary data processing, improve efficiency, and reduce power consumption.

As shown in Fig. 2, the device may further include a man-machine interaction module 25. The man-machine interaction module 25 may be a touch screen, a voice module, or a button, and is set as receiving the input of the user to conduct information input, or start up the alarm module 24 to conduct alarm for help or relieve the alarm for help according to the action of the user, or may play the prompt by voice, for example inputting the basic information of the user through the touch screen, or implementing one key alarm through the button. In this way, the user can conduct the slump warning in time when the detection result is affected by problems of insufficient sampling rate and algorithm recognition rate, and a timely risk prompt is played to the user when the user is detected to have the slump risk, which is very convenient.

Preferably, in order to meet the requirement of the user more human-friendly, and perform real-time human-body detection and timely prompt to avoid the occurrence of slump, a solution on preventing the slump is further provided according to one embodiment of the invention to meet the requirement of informing the user to have a rest or take other prevention measures under the condition that the human body of the user is abnormal.

As shown in Fig. 2, the signal processing module 20 further includes a slump prevention unit 203, and is configured to perform analysis processing according to the human-body posture behavior, human-body biometrics data, and environmental data collected by the signal collection module 21, so as to detect the body state of human body and calculate the slump risk grade, and the risk prompt information is outputted according to the slump risk grade to prompt the user to conduct corresponding processing in time, such as having a rest, taking medicine, etc. The signal collection module 21 collects the human-body posture behavior data through the sensor configured to collect the posture and behavior data of the human body, collects the human-body biometrics data through the sensor configured to collect the biological sign data of the human body, and collects the environmental data through the sensor configured to collect the environmental data from a vicinity of the human body. The sensor configured to collect the human-body posture behavior data includes a tri-axis acceleration sensor, a gyroscope, and an air pressure altitude sensor, which are used for recognizing whether the human body behavior and posture is abnormal. The sensor configured to collect the human-body biometrics data includes one of or a combination of more than two of a heart rate sensor, a photoelectric blood pressure sensor, a temperature sensor, a humidity sensor, and an oxygen saturation sensor, which are used for judging whether the body state of the wearer is good. The sensor configured to collect the environmental information data from a vicinity of the human body includes one of or a combination of more than two of an air pressure sensor, a temperature sensor, a humidity sensor, and an air velocity sensor, which are used for judging whether the current environment is a slump prone environment.

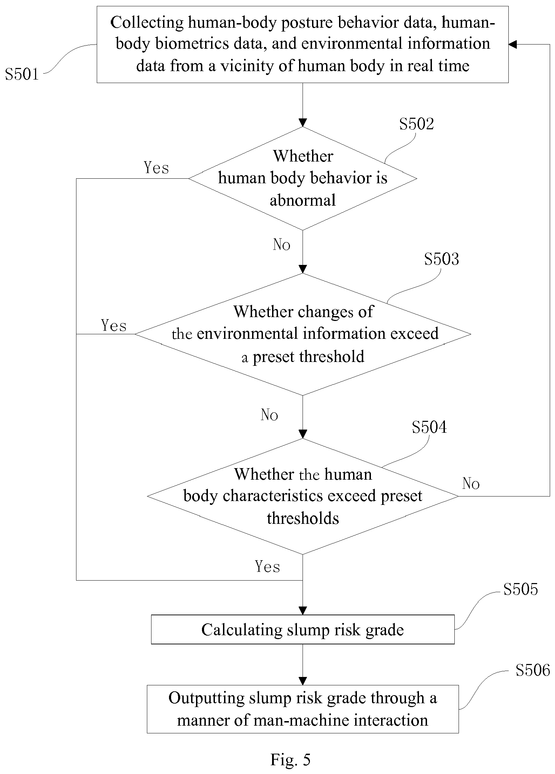

Fig. 5 schematically illustrates a method for preventing and monitoring human body slump, according to one embodiment of the present invention. Taking the sensor configured to collect the human-body posture behavior data including a tri-axis acceleration sensor, a gyroscope, and an air pressure altitude sensor, the sensor configured to collect the human-body biometrics data including a heart rate sensor, a humidity sensor, a photoelectric blood pressure sensor, and an oxygen saturation sensor, and the sensor configured to collect the environmental information including a temperature sensor and a pressure sensor for example, and as shown in Fig. 5, the method for preventing and monitoring human body slump includes the following steps.

In step S501, the human-body posture behavior data, biometrics data, and environmental data are collected in real time.

The human-body posture behavior data is collected through the tri-axis acceleration sensor, the gyroscope, and the air pressure altitude sensor in real time, which includes the tri-axis acceleration data, the angular velocity data, and the air pressure altitude data. The biometrics data is collected through the heart rate sensor, the humidity sensor, the photoelectric blood pressure sensor, and the oxygen saturation sensor, which includes the heart rate data, the humidity data on body surface, the blood pressure data, and the oxygen saturation data. The environment temperature data and the air pressure data are collected in real time through the temperature sensor and the air pressure sensor.

In step S502, whether the human body is abnormal is judged.

Whether the human body is in movement (for example, being walking) is recognized according to the tri-axis acceleration data or the angular velocity data of the gyroscope, and it is prior art to judge whether the human body is in movement through the acceleration sensor and the gyroscope, thus will not be elaborated herein. For under a normal condition, people walks very uniformly, thus when the people is judged to be in movement, the abnormal state can be determined by judging whether the human body is "staggered" (i.e., stop-and-go walking) according to the step counting function of the tri-axis acceleration sensor or the gyroscope, and whether the human body is abnormal of "unsteady center of gravity" according to the changes of the tri-axis acceleration data and the air pressure altitude data. If the abnormity occurs, the slump alarm flag SLUMP_WARN_FLAG is set as TRUE. It should be illustrated that the judgment whether the human body is staggered and has abnormal states of being staggered and unsteady center of gravity may be implemented through using the existing function of each of the acceleration sensor above. Therefore, the implementation process will not be elaborated herein. When one of or a combination of more than two of the human body abnormal states above occurs, the human body behavior is judged to be abnormal, then the slump alarm flag SLUMP_WARN_FLAG is set as TRUE, and the calculation to the slump risk grade of Step S505 is then executed. If the abnormity does not occur (i.e., FALSE), the judgment of step S503 is further executed.

In step S503, whether the environmental change exceeds a given threshold is judged.

Whether it is a slump prone environment currently is judged according to collected surrounding environment temperature data and air pressure data. Common environmental factors leading to the slump includes: rapid temperature change, air pressure change, etc. The difference of the temperature data and the air pressure data obtained in two times before and after is calculated through saving the collected temperature data and air pressure data in a period of time (such as in 4s), so as to judge whether the temperature difference value and the air pressure value (i.e., temperature change and air pressure change) are greater than given thresholds TEMPERATURE_WARN_TH and AIRPRESS_WARN_TH. If at least one of the two difference values satisfies the condition, then the environment is judged to be a slump prone environment, the slump alarm flag SLUMP_WARN_FLAG is set as TRUE, and the calculation of the slump risk grade in step S505 is conducted. Otherwise, the judgment in step S504 is conducted. For example, it is provided that the temperature datum collected successively in a period time are respectively $T_n$ and $T_{n-1}$, the air pressure datum are respectively $P_n$ and $P_{n-1}$, and then the temperature change and the air pressure change may be obtained through TEMPERATURE_CHANGE= $T_n$-$T_{n-1}$ and AIRPRESS_CHANGE= $P_n$-$P_{n-1}$, so as to judge whether TEMPERATURE_CHANGE>TEMPERATURE_WARN_TH and AIRPRESS_CHANGE>AIRPRESS_WARN_TH are satisfied. If one of the two changes meets the condition, the slump alarm flag is set as TRUE, and the slump rick grade calculation is conducted.

In step S504, whether the human-body biometrics data exceeds a given threshold is judged.

Whether the current body state is good is judged according to human-body biometrics data collected including: the heart rate data, the humidity data on body surface, the blood pressure data, and the oxygen saturation data. Under a normal condition, before the occurrence of slump, the elderly may have the characteristics of "abnormal sweating", "accelerated heart beat", "increased blood pressure", etc. The biometrics data above is obtained through the sensor to judge whether the data is greater than the upper limit threshold set in advance or is smaller than the lower limit threshold set in advance, so as to recognize the body state of human body, for example, judging that whether the humidity data HUMIDITY>HUMIDITY_WARN_TH, the heart rate data HEART_RATE>HEART_RATE_HIGH_WARN_TH or HEART_RATE<HEART_RATE_LOW_WARN_TH, the blood pressure data BLOODPRESSURE>BLOODPRESSURE_HIGH_WARN_TH or BLOODPRESSURE<BLOODPRESSURE_HIGH_WARN_TH, and the oxygein saturation data SPO<SPO_TH are satisfied. If one of or a combination of more than two of the conditions above is satisfied, then the current body state of the wearer is judged to be unwell and have slump risk, and the slump alarm flag SLUMP_WARN_FLAG is set as TRUE, and the calculation of the slump risk in step S505 is executed. Otherwise, the data collection in step S501 is performed repeatedly.For example, under a normal condition, the heart beats are [60,100], and when the collected heart beat data HEART_RATE exceeds 100, the abnormity may be considered. For another example, the normal condition of blood pressure is that diastolic pressure<85/systolic pressure<130, and if collected blood pressure data exceeds the scope, the body abnormity of high blood pressure is considered. For another example, the oxygen saturation is 90% above under a normal condition, and if the oxygen saturation data collected is smaller than the value, then it is possible to cause disease. In practical application, the threshold setting can be selected according to the basic medical science, and it can also be set by performing off-line sample training according to basic information of the user, such as the age, sex, medical history, etc. in this case the threshold is set according to the training result.

In step S505, the slump risk grade is calculated.

The value of the slump alarm flag SLUMP_WARN_FLAG is read to judge, when judging that the slump alarm flag SLUMP_WARN_FLAG is TURE, then the biometrics data of the human body collected currently is read to perform weighted calculation, so as to obtain the slump risk grade. Specifically, the human-body biometrics data is obtained, corresponding weighted value is distributed for the obtained biometrics data of the human body, and the current slump risk grade of the wearer is calculated according to a formula $score = \sum_{i=0}^{n} a_i * |D_i - DH_i|$ , wherein $a_i$ is the weighted value of $i^{th}$ biometrics data (the sum of all weighted value of n biometrics data is 1), $D_i$ is the value of $i^{th}$ biometrics data collected, $DH_i$ is the value of $i^{th}$ biometrics data under a normal condition, n is the number of the sensor configured to collect the biometrics data in the signal collection module. For example, the sensor configured to collect the biometrics data is numbered as follows: the sensor is the humidity sensor when n=0, the sensor is the heart rate sensor when n=1, the sensor is the blood pressure sensor, when n=2, and the sensor is the oxygen saturation sensor when n=3, and in this case, $a_o$ is the weighted value of the body humidity, $a_1$ is the weighted value of the heart rate, etc. Moreover, $D_0$, $D_1$, $D_2$, and $D_3$ are respectively collected current humidity data, heart beat data, blood pressure data, oxygen saturation data, and in a similar way, $DH_0$, $DH_1$, $DH_2$, and $DH_3$ are respectively the values of humidity of human body, heart beat, blood pressure, and oxygen saturation under a normal condition. Therefore, by distributing a weighted value and setting the standard value for each item of the biometrics data, the slump risk grade (referred to as score) of current user is calculated according to the biometrics data collected currently, and by judging the slump risk grade value, the risk degree of the occurrence of slump is obtained, that is, the greater the value is, the higher the slump risk degree is.

In order to improve the accuracy of calculated slump risk grade, the calculation may be performed in the combination of basic personal information of a wearer, such as in the combination of the age, height, and weight, and in this case the calculation can be performed by formula

$$score = (w_0 * \max(1, \frac{age}{age_0}) + w_1 * \frac{weight}{height * weight}) * \sum_{i=0}^{n} a_i * |D_i - DH_i|$$

. In this formula, $w_o$ and $w_1$ are weighted values, the sum of the two weighted values is 1, and $age_0$ is given standard age value. When the real age exceeds the standard age, the degree of slump risk may increase with the increase of the real age. Moreover, the ratio of weight and height is an obesity index, wherein it is usually considered that the greater the index is, the poor the condition of human health is, so that the slump risk of human body may increase with the increase of the obesity index.

It should be illustrated that the evaluation of each weight according to the embodiment above may be correspondingly adjusted with reference to practical application and personal situation of the wearer, as long as the sum of each weight coefficient is 1, that is, the sum of each $age_0$ is 1 and the sum of $w_o$ and $w_1$ is 1. In specific application, under the condition that the sum of the weight is unchanged, the weight may be adjusted in the combination of personal health condition of the wearer, for example, the weighted value of heart rate data is properly increased for the user with cardiopathy history, the weighted value of blood pressure data is properly increased for the user with hypertension history, etc.

In step S506, the slump risk grade is outputted through a man-machine interaction manner.

Corresponding risk prompt is set according to an evaluation interval of the slump risk grade. The calculated value of slump risk grade outputted is read to judge, and corresponding risk prompt is outputted to the user through the man-machine interaction module according to the calculated value thereof. For example, "0" is set as representing having no risk, and the corresponding voice prompt is not set; "5" is set as high risk, the voice prompt "you have physical complaints, please have a rest soon" is set, in this case when judging that the outputted slump risk grade is "5", the voice prompt and the similar are played through the man-machine interaction module.

The slump risk of the user may be monitored through the human-body posture behavior data, biometrics data and environmental data according to the embodiment above, and when the user has the slump risk, the prompt is conducted in time through the man-machine interaction module, so as to prevent the occurrence of the slump, and provide more effective guardianship for the user. The embodiment calculates the value of the slump risk grade in a weighted manner in the combination of the human-body biometrics data and the basic personal information of the user and outputs the same, so as to quantize the slump risk of the user, which can monitor the slump risk according to body characteristics of the user collected in real time, prevent the occurrence of risk, and have higher accuracy and stronger suitability .

Alternatively, the alarm module 25 may further be a loud speaker play device, thus when the help mode is started, as the help message is sent to the guardian through the wireless communication module 23, the loud speaker is also started up to play a voice distress signal meanwhile, so that the user can get help in time.

The device for monitoring and preventing slump provided by the present invention can be conveniently worn on the waist of the user as a waist belt, which is very convenient. Moreover, the device of the present invention performs human body slump detection through the human-body posture behavior data and human-body biometrics data, which is further in conformity with the behavior characteristics of the user, and has higher accuracy. Meanwhile, the device of the present invention provides a device wearing state detection function, which can avoid the badness of misinformation caused by the device that is not worn or is worn incorrectly, so as to further improve the accuracy of the slump detection, thus the slump help information and the position information of the wearer are sent to the guardian timely and accurately. The device of the present invention further provides a prevention monitoring function at the same time, which can perform slump risk analysis and calculation in time when the body of the user is abnormal or the environment becomes the slump prone environment, and prompt the degree of slump risk possible to occur in a man-machine interaction manner for the user to prevent the occurrence of the slump, so that a more comprehensive guardianship to the user is achieved. The device of the present invention can further implement the information interaction with the user through the touch screen, the button, the voice recognition, etc. which facilitates the operation of the user, thus can meet the requirement of calling for help or relieving the help through the button when the risk or misinformation occurs, so that the user experience is better.

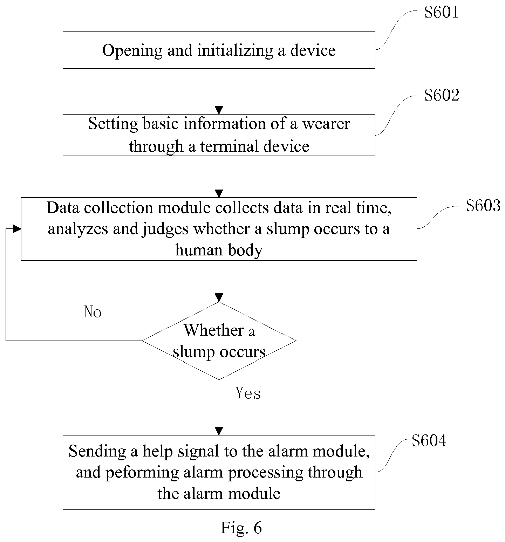

Fig. 6 schematically illustrates an operation method of a device for monitoring and preventing slump, according to one embodiment of the present invention. As shown in Fig. 6, the method includes the following steps.

In step S601, the device is opened and initialized.

The user opens a power supply of the device, waits for the device to perform the initialization of the data automatically, which comprises giving an initial value to state variables of the device, for example, the wearing state flag WEAR_FLAG is initialized as FALSE, the slump state flag SLUMP_FLAG is assigned as FALSE, the slump alarm flag SLUMP_WARN_FLAG is set as FALSE, the slump risk grade is set as "0", etc.

In step S602, basic information of a wearer is set through a terminal device.

The basic personal information, such as the sex, age, weight, height, medical history, etc., of the wearer of the device is set through a user platform on the terminal device including the mobile phone, computer, etc. and the set basic personal information of the user is sent to the corresponding device for temporary storage through wireless communication manners including the bluetooth, wifi, etc.

In step S603, a signal collection module collects data in real time, analyzes and judges whether a slump occurs to the human body.

The signal collection module starts to collect the human-body posture behavior data and human-body biometrics data through the sensor, and a signal processing module performs the analysis according to collected human-body posture behavior data and human-body biometrics data, so as to judge whether the slump occurs to the human body. When judging that a slump occurs, then step S604 is executed to send a help signal to the alarm module. Otherwise, the data collection operation in step S603 is conducted continuously. The detail for how the signal processing module performing the analysis according to collected human-body posture behavior data and human-body biometrics data to judge whether the slump occurs to the human body can be found in the text above (seeing the description about the method of Fig. 3), thus it will not be elaborated herein.

In step S604, the help signal is sent to the alarm module, and alarm processing is performed by the alarm module.

The signal processing module sends the signal for help to the alarm module (such as character "1"), and obtains the geographical location information of the user through the positioning module at the same time. The geographical position information of the user and the help content are sent to the remote terminal device of the guardian through the wireless communication module after the alarm module receives the help signal, so as to inform the guardian, so that the user can get timely help.

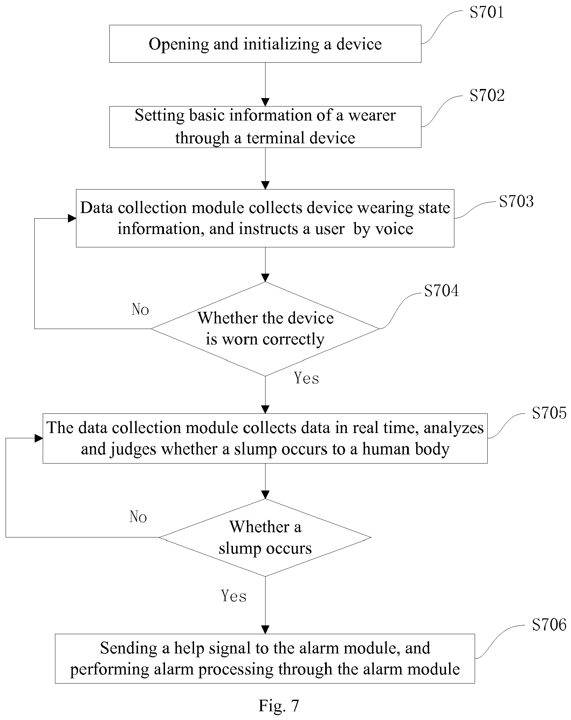

Fig. 7 schematically illustrates an operation method of a device for monitoring and preventing slump, according to another embodiment of the present invention. As shown in Fig. 7, the difference between the embodiment and the embodiment as shown in Fig. 6 lies in that the embodiment firstly needs to detect whether the device is worn correctly, and whether a slump occurs to the human body is detected only when the wearing is correct. The details are as follows.

In step S701, the device is opened and initialized.

In step S702, the basic information of the wearer is set through a terminal device.

In step S703, the signal collection module collects wearing state information data, and instructs the user by voice.

The information collection module collects the wearing state information through the sensor configured to collect device wearing state information data, and when the pressure data is detected to increase gradually, the wearing steps are played by voice.

In step S704, whether the device is worn correctly is judged.

The signal processing module detects whether the device is worn correctly according to collected wearing state information, and when the device is detected to wear correctly, step S705 is processed. Otherwise, the operations that the collection of the device wearing sate information data and the voice play in step S703 are conducted continuously. Refer to the description of Fig. 4 in the text above for the implementation process of specific detection on whether the device is worn correctly, which will not be elaborated herein.

In step S705, the information collection module collects data in real time, analyzes and judges whether the slump occurs to the human body.

In step S706, a help signal is sent to the alarm module, and alarm processing is conducted through the alarm module.

Refer to step S601 to step S604 in the text above for the specific implementation processes of step S701, step S702, step S705 and step S706. Slump detection may be executed under the premise of correct wearing according to the embodiment, which can improve the efficiency and accuracy of the detection.

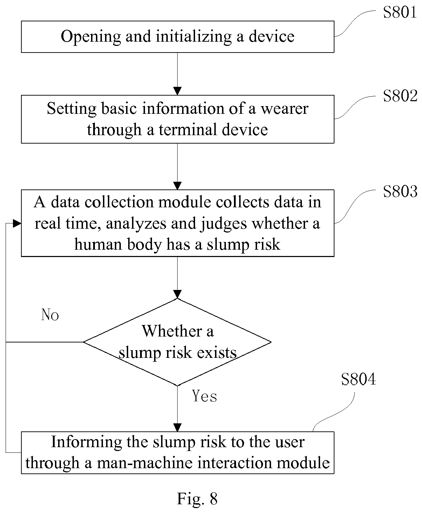

Fig. 8 schematically illustrates an processing method of an intelligent human body slump monitoring device, according to another embodiment of the present invention. As shown in Fig. 8, the difference between the embodiment and the embodiment as shown in Fig. 6 lies in that the embodiment analyzes and judges the slump risk of the human body according to collected data, so as to reach to the object of preventing the occurrence of slump, and provide more intelligentized and more convenient protection for the user. The details are as follows.

In step S801, the device is opened and initialized.

In step S802, the basic information of the wearer is set through a terminal device.

In step S803, the information collection module collects data in real time, analyzes and judges whether the slump occurs to the human body.

The information collection module collects the human-body posture behavior data, human-body biometrics data, and environmental information data from a vicinity of human body in real time, and the signal processing module performs the analysis according to collected data, so as to judge whether the human body has the slump risk. When judging that there is a slump risk, the slump risk grade is calculated and outputted. Wherein, refer to the description of Fig. 5 in the text above for the specific implementation process of judging whether the human body has the slump risk, and calculating and outputting the slump risk grade when judging that the human body has a slump risk, which will not be elaborated herein.

In step S804, a slump risk is informed to the user through a man-machine interaction module.

The signal processing module informs the slump risk to the user through the man-machine interaction module according to the slump risk grade calculated so as to prompt the user to conduct risk prevention and corresponding process in time. For example, when the slump risk grade calculated by the signal processing module is "5", then the prompt content corresponding to the risk grade is sent to the man-machine interaction module to be played by voice.

In this embodiment, step S801 and step S802 are the same as step S601 and step S602. The body state and slump risk can be prompted to the user in time by analyzing and calculating the slump risk grade of the human body, so that timely prompt and help for the user can be provided when the slump is possible to occur, thus the incidence rate of slump of the user is reduced, and the degree of safety of the user is improved.

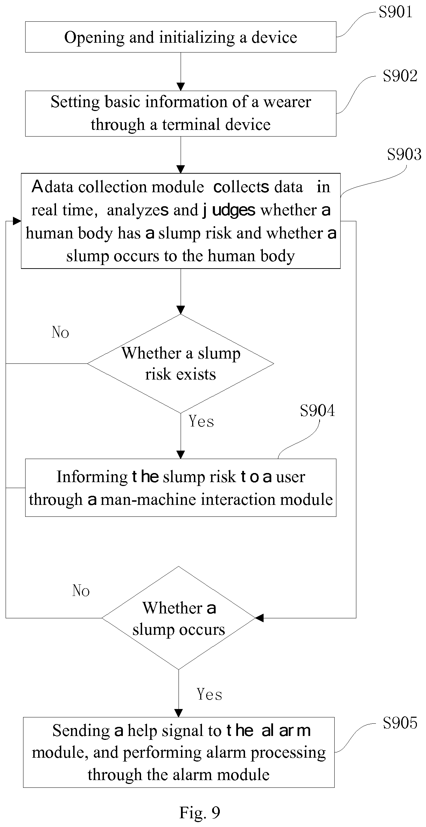

Fig. 9 schematically illustrates an operation method of a device for monitoring and preventing slump, according to another embodiment of the present invention. As shown in Fig. 9, the method includes the following steps.

In step S901, the device is opened and initialized.

In step S902, basic information of the wearer is set through a terminal device.

In step S903, an information collection module collects data in real time, analyzes and judges whether the human body has a slump risk and whether a slump occurs to the human body.

The information collection module collects the human-body posture behavior data, human-body biometrics data and environmental information data surrounding the human body in real time, and the signal processing module conducts analysis according to collected human-body posture behavior data and human-body biometrics data to judge whether the slump occurs to the human body. Meanwhile, the analysis is conducted according to collected human-body posture behavior data, human-body biometrics data and environmental information data to judged whether the human body has the slump risk, and when judging that the human body has a slump risk, the slump risk grade is calculated to output. Step S905 is executed when the slump occurs, and step S904 is executed when the slump does not occur but there is a slump risk. Wherein, refer to the description of Fig. 3 in the text above to judge whether the slump occurs to the human body, and refer to the description of Fig. 5 in the text above for the specific implementation process of judging whether the human body has the slump risk, and calculating the slump risk grade and outputting same when judging that the human body has a slump risk, which will not be elaborated herein.

In step S904, a slump risk is informed to the user through a man-machine interaction module.

In step S905, a help signal is sent to the alarm module, and alarm processing is performed by the alarm module.

Wherein, refer to the corresponding description in the text above for the specific implementation processes of steps S901, S902, S904, and S905. Both the slump monitoring and the slump prevention to the user can be achieved in the embodiment. The slump monitoring is determined through the judgment of slump state flag SLUMP_FLAG, and the slump prevention is determined through the slump risk flag SLUMP_WARN_FLAG and the slump level, so that it can provide a double protection for the user, which can effectively guarantee the security of the user.

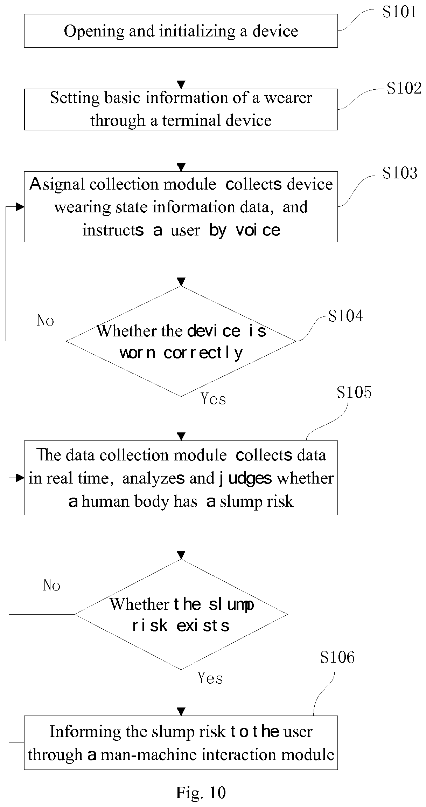

Fig. 10 schematically illustrates an operation method of a device for monitoring and preventing slump, according to another embodiment of the present invention. As shown in Fig. 10, the difference between the embodiment and the embodiment as shown in Fig. 8 lies in that the embodiment firstly needs to detect whether the device is worn correctly, and the detection whether the human body has the slump risk is performed only if the device is worn correctly. The details are as follows.

In step S101, the device is opened and initialized.

In step S102, the basic information of the wearer is set through a terminal device.

In step S103, the signal collection module collects device wearing state information, and instructs the user by voice.

The information collection module collects the wearing state information through the sensor configured to collect device wearing state information data, and when the pressure data is detected to increase gradually, the wearing steps are played by voice.

In step S104, whether the device is worn correctly is judged.

The signal processing module detects whether the device is worn correctly according to collected wearing state information. When the device is detected to wear correctly, the Step S105 is processed. Otherwise, the collection of the device wearing state information and the voice play of Step S103 are performed repeatedly. Wherein, refer to the description of Fig. 4 in the text above for the implementation process of specific detection whether the device is worn correctly, which will not be elaborated herein.

In step S105, the information collection module collects data in real time, analyzes and judges whether the human body has slump risk.

In step S106, a help signal is sent to the alarm module, and alarm processing is performed be the alarm module.

Wherein, refer to step S801 to step S804 in the text above for the specific implementation processes of step S101, step S102, step S105 and step S106. The slump risk will only be analyzed under the premise that the device is worn correctly through the embodiment, which can improve the efficiency and accuracy of the detection.

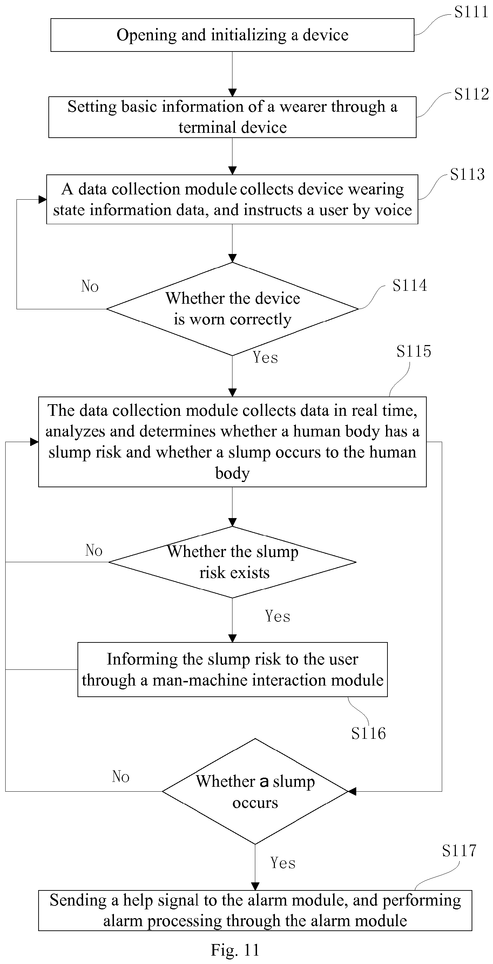

Fig. 11 schematically illustrates an operation method of a device for monitoring and preventing slump, according to another embodiment of the present invention. As shown in Fig. 11, the method includes the following steps.

In step S111, the device is opened and initialized.

In step S112, basic information of a wearer is set through a terminal device.

In step S113, a signal collection module collects device wearing state information, and instructs the user by voice.

The information collection module collects the wearing state information through the sensor configured to collect device wearing state information data, and when the pressure data is detected to increase gradually, the wearing step is played by voice.

In step S114, whether the device is worn correctly is judged.

The signal processing module detects whether the device is worn correctly according to collected wearing state information. When detecting that the device is worn correctly, step S 115 is executed. Otherwise, the operations that collecting the device wearing state information and playing the voice in step S 113 are performed continuously. Wherein, refer to the description of Fig. 4 in the text above for the implementation process of specific detection on whether the device is worn correctly, which will not be elaborated herein.

In step S115, the information collection module collects data in real time, analyzes and judges whether the human body has a slump risk and whether a slump occurs to the human body.

The information collection module collects the human-body posture behavior data, human-body biometrics data and environmental information from a vicinity of human body in real time, and the signal processing module performs analysis according to collected human-body posture behavior data and human-body biometrics data to judge whether the slump occurs to the human body. Meanwhile, the analysis is performed according to collected human-body posture behavior data, human-body biometrics data, and environmental information to judge whether the human body has the slump risk, and when judging that the human body has a slump risk, the slump risk level is calculated and outputted. Step S117 is executed when the slump occurs, and step S116 is executed when the slump does not occur but there is slump risk. Wherein, refer to the description of Fig. 3 in the text above to judge whether the slump occurs to the human body, refer to the description of Fig. 5 in the text above for the specific implementation process of judging whether the human body has the slump risk, and calculating and outputting the slump risk grade when judging that the human body has a slump risk, which will not be elaborated herein.

In step S116, a slump risk is informed to the user through a man-machine interaction module.

In step S 117, a help signal is sent to the alarm module, and alarm processing is performed by the alarm module.

Wherein, refer to the corresponding description in the text above for the specific implementation processes of steps S111, S112, S116, and S117. Both the slump monitoring to the user and the slump prevention to the user can be achieved according to the embodiment. The slump monitoring is implemented by means of the judgment of slump state flag SLUMP_FLAG, and the slump prevention is determined through the slump risk flag SLUMP_WARN_FLAG and the slump grade, so that a double protection for the user is provided, which can effectively guarantee the security of the user.

Preferably, in order to avoid the badness led by misinformation when the detection is wrong and better guarantee the personnel safety of the wearer, the processing method of the present invention may further includes: receiving the signal input of the user through the button, playing/stopping the loud speaker alarm, and sending help information/help relieving information to the remote terminal. For example, the device is provided with a button, if the user presses the button briefly, then the signal processing module receives the input of the user to send the help signal to the alarm module, and the alarm module plays the loud speaker and sends the help information containing the position information to remote terminal of the guardian. If the user presses the button for a long time, then the signal processing module receives the input of the user to send the help relieving signal to the alarm module, and the alarm module stops playing the voice for help of the loud speaker and sends the information that the risk is relieved to the guardian.

The human body slump state detection is implemented through the present invention, which has higher accuracy, and can meet the requirement on the slump monitoring to the elderly patient. Meanwhile, the method according to the present invention further provides the detections on whether the device is worn correctly and whether there is a slump risk , which can avoid the misinformation led by the device wearing problem, and can inform to do processing in time when the slump risk occurs, so that the object of preventing the occurrence of slump is achieved. The device and method provided can be more intelligent and convenient, and have higher accuracy in detection.

The contents above are merely some embodiments of the present invention. Those skilled in the art may also figure out a plurality of deformations and improvements without departing from the conception of the present invention, which shall all fall within the protection scope of the present invention.

**Claims**

1. A device for monitoring and preventing slump, including a positioning module, a wireless communication module, an alarm module, a signal collection module, and a signal processing module, wherein
the signal collection module is used for collecting human-body posture behavior data and human-body biometrics data;
the signal processing module comprises a slump detection unit, which is configured to perform analysis processing according to the human-body posture behavior data and the human-body biometrics data to determine a state of the human body, and output a signal for help to the alarm module when determining that a slump occurs to the human body; and
the alarm module generates a help message according to the signal and outputs same.

2. The device according to claim 1, wherein the signal processing module further comprises a wearing state detection unit, the signal collection module is further used for collecting wearing state information of the device, and the wearing state detection unit is configured to perform analysis processing according to the wearing state information and output a wearing state flag; and
the slump detection unit performs human body slump detection and outputs the signal to the alarm module according to the outputted wearing state flag.

3. The device according to claim 1 or 2, wherein the signal collection module is further used for collecting environmental information from a vicinity of the human body, and
the signal processing module further comprises a slump prevention unit configured to judge the state of the human body according to the human-body posture behavior data, human-body biometrics data and the environmental information from a vicinity of the human body, calculate a slump risk grade in a weighted manner according to the judgment, and output the slump risk grade.

4. The device according to claim 3, wherein, the positioning module collects location information of the device, and the alarm module sends a help information containing the location information to a remote terminal through the wireless communication module.

5. The device according to claim 4, further comprising a man-machine interaction module, wherein the man-machine interaction module comprises one of or a combination of more than two of a touch screen, a button and a voice player, and the slump prevention unit outputs the slump risk grade by way of the man-machine interaction module.

6. A processing method of a device for monitoring and preventing slump, wherein the device comprises a positioning module, a wireless communication module, an alarm module, a signal collection module, and a signal processing module, and the processing method comprises:

   collecting, by the signal collection module, human-body posture behavior data and human-body biometrics data in real time, and outputting the same to the signal processing module;
   performing, by the signal processing module, analysis processing according to the human-body posture behavior data and human-body biometrics data, determining a state of the human body, and when determining that a slump occurs to the human body, outputting a help signal to the alarm module; and
   sending, by the alarm module, a help message containing location information to a remote terminal according to the help signal.

7. The method according to claim 6, wherein the processing method further comprises:

   collecting, by the signal collection module, wearing state information of the device in real time, and outputting the wearing state information to the signal processing module;
   analyzing, by the signal processing module, according to the wearing state information, judging the wearing state of the device, and outputting a wearing state flag according to the wearing state of the device; and
   reading, by the signal processing module, the wearing state flag to judge, when judging that the wearing state flag indicates that the device is worn correctly, determining a slump state of the human body and outputting the help signal to the alarm module according to the slump state.

8. The method according to claim 6, wherein the signal collection module collects the human-body posture behavior data through a tri-axis acceleration sensor, and

the operation that performing, by the signal processing module, analysis processing according to the human-body posture behavior data and human-body biometrics data, and determining the state of the human body is performed by:

reading tri-axis acceleration data collected by the tri-axis acceleration sensor, and calculating a sum ACC_SUM of changes of the tri-axis acceleration data;

judging whether the sum ACC_SUM of changes of the tri-axis acceleration data is smaller than a given threshold, and setting a body posture flag as TRUE if the condition is satisfied; and

reading the human-body biometrics data, and judging whether the human-body biometrics data is greater than the upper limit of given thresholds or is smaller than the lower limit of given threshold, judging a severe degree of the slump of the human body according to a level of given thresholds if the condition is satisfied, and outputting a human body slump flag.

9. The method according to claim 6, wherein the processing method further comprises:

collecting, by the signal collection module, environmental information from a vicinity of the human body in real time, and outputting the environmental information to the signal processing module; and

performing, by the signal processing module, analysis processing according to the human-body posture behavior data, human-body biometrics data and environmental information, judging the state of the human body, calculating the data in a weighted manner and outputting a slump risk grade according to the judgment.

10. The method according to claim 9, wherein the operation that performing, by the signal processing module, analysis processing according to the human-body posture behavior data, human-body biometrics data and environmental information, judging the state of the human body, calculating the data in a weighted manner and outputting the slump risk grade according to the judgment, is performed by:

reading the human-body posture behavior data, human-body biometrics data and environmental information, judging whether the datum are abnormal respectively, and setting the slump risk flag as TRUE when one of the datum are abnormal; and

reading a value of a slump alarm flag SLUMP_WARN_FLAG to judge, when the slump alarm flag SLUMP_WARN_FLAG is TURE, obtaining the human-body biometrics data and calculating a current slump risk grade according to a formula $score = \sum_{i=0}^{n} a_i * |D_i - DH_i|$

$$score = (w_0 * \max(1, \frac{age}{age_0}) + w_1 * \frac{weight}{height * weight}) * \sum_{i=0}^{n} a_i * |D_i - DH_i|;$$

wherein $a_i$ is a weighted value of $i^{th}$ biometrics data, $D_i$ is a value of $i^{th}$ biometrics data collected, $DH_i$ is a value of $i^{th}$ biometrics data under a normal condition, n is a number of a sensor configured to collect the biometrics data in the signal collection module, age is age, height is height, weight is weight, $w_0$ and $w_1$ are weighted values, and $age_0$ is a given standard age value.

Fig. 1

Fig. 2

CollectIng human-body posture behavior data in real time — S301

Whether the human body enters a "peaceful" period — S302

No

Yes

Collecting human-body biometrics data in real time — S303

Whether the human-body biometrics data is abnormal — S304

No

Yes

Classifying a current slump severe degree of a user, and informing the user to confirm abnormality by voice — S305

Whether the user presses a button

Yes

No

Judging that a slump occurs, and sending a corresponding help signal according to a slump level flag to the alarm module — S306

Fig. 3

Collecting temperature, human body bioelectricity signal, pressure, and acceleration data in real time, and performing wearing instruction by voice to a user — S401

Comparing the temperature T1 of the human body side with an air temperature T2 to determine wHether T2-T1>TH is satisfied — S402

No

Whether the human body bioelectricity signal is high level? — S403

No

Yes

Yes

Whether the value of each pressure sensor satisfies P1=P2= ... =PN>O? — S404

No

Yes

S406

Prompting the user that the wearing direction is wrong by voice

Whether satisfies AY=g and AX=AZ=0 while in a normal standing situation? — S405

No

Yes

Judging that the wearing is correct, and set a correct wearing state flag as TRUE — S407

Fig. 4

Collecting human-body posture behavior data, human-body biometrics data, and environmental information data from a vicinity of human body in real time

S501

S502

Whether human body behavior is abnormal

Yes

No

S503

Whether changes of the environmental information exceed a preset threshold

Yes

No

S504

Whether the human body characteristics exceed preset thresholds

No

Yes

S505

Calculating slump risk grade

S506

Outputting slump risk grade through a manner of man-machine interaction

Fig. 5

Opening and initializing a device — S601

Setting basic information of a wearer through a terminal device — S602

Data collection module collects data in real time, analyzes and judges whether a slump occurs to a human body — S603

Whether a slump occurs

No

Yes

Sending a help signal to the alarm module, and peforming alarm processing through the alarm module — S604

Fig. 6

S701

Opening and initializing a device

S702

Setting basic information of a wearer
through a terminal device

S703

Data collection module collects device wearing state
information, and instructs a user by voice

S704

No — Whether the device
is worn correctly

Yes

S705

The data collection module collects data in real time, analyzes
and judges whether a slump occurs to a human body

No — Whether a
slump occurs

Yes

S706

Sending a help signal to the alarm module, and
performing alarm processing through the alarm module

Fig. 7

Opening and initializing a device — S801

Setting basic information of a wearer through a terminal device — S802

A data collection module collects data in real time, analyzes and judges whether a human body has a slump risk — S803

Whether a slump risk exists

No

Yes

Informing the slump risk to the user through a man-machine interaction module — S804

Fig. 8

```
┌─────────────────────────────────────┐      ⌐ S901
│   Opening and initializing a device  │
└─────────────────────────────────────┘
                    │
                    ▼
┌─────────────────────────────────────┐      ⌐ S902
│  Setting basic information of a wearer│
│         through a terminal device     │
└─────────────────────────────────────┘
                    │                                S903
                    ▼
┌─────────────────────────────────────┐
│ A data collection module collects data in │
│  real time, analyzes and judges whether a  │
│  human body has a slump risk and whether a │
│    slump occurs to the human body          │
└─────────────────────────────────────┘
                    │
                    ▼
    No         ◇ Whether a slump ◇
    ┌──────────    risk exists
    │               │
    │              Yes                        S904
    │               ▼
    │   ┌─────────────────────────────────────┐
    │   │ Informing the slump risk to a user   │
    │   │ through a man-machine interaction module │
    │   └─────────────────────────────────────┘
    │
    │   No       ◇ Whether a ◇
    │   ┌────────  slump occurs
    │   │             │
    │                Yes
    │                 ▼
    │   ┌─────────────────────────────────────┐   ⌐ S905
    │   │ Sending a help signal to the alarm    │
    │   │ module, and performing alarm processing│
    │   │      through the alarm module          │
    │   └─────────────────────────────────────┘
```

Fig. 9

Opening and initializing a device — S101

Setting basic information of a wearer through a terminal device — S102

A signal collection module collects device wearing state information data, and instructs a user by voice — S103

Whether the device is worn correctly — S104

No

Yes

The data collection module collects data in real time, analyzes and judges whether a human body has a slump risk — S105

Whether the slump risk exists

No

Yes

Informing the slump risk to the user through a man-machine interaction module — S106

Fig. 10

Opening and initializing a device ⟶ S111

Setting basic information of a wearer through a terminal device ⟶ S112

A data collection module collects device wearing state information data, and instructs a user by voice ⟶ S113

Whether the device is worn correctly ⟶ S114

No

Yes

The data collection module collects data in real time, analyzes and determines whether a human body has a slump risk and whether a slump occurs to the human body ⟶ S115

Whether the slump risk exists

No

Yes

Informing the slump risk to the user through a man-machine interaction module ⟶ S116

Whether a slump occurs

No

Yes

Sending a help signal to the alarm module, and performing alarm processing through the alarm module ⟶ S117

Fig. 11

# INTERNATIONAL SEARCH REPORT

| | International application No. |
|---|---|
| | **PCT/CN2016/084730** |

**A. CLASSIFICATION OF SUBJECT MATTER**

G08B 21/04 (2006.01) i

According to International Patent Classification (IPC) or to both national classification and IPC

**B. FIELDS SEARCHED**

Minimum documentation searched (classification system followed by classification symbols)

G08B

Documentation searched other than minimum documentation to the extent that such documents are included in the fields searched

Electronic data base consulted during the international search (name of data base and, where practicable, search terms used)

CNABS; CNTXT; VEN: paralyzation detection, paralyzation monitor, paralyzation prevention, fall prevention, monitor, detect, device wear, equipment wear, wearing state, wearing detection, paraly+, fall+, grac+, slow+, wearable, worn

**C. DOCUMENTS CONSIDERED TO BE RELEVANT**

| Category* | Citation of document, with indication, where appropriate, of the relevant passages | Relevant to claim No. |
|---|---|---|
| PX | CN 105118236 A (GUANGDONG APPSCOMM CO., LTD.), 02 December 2015 (02.12.2015), claims 1-10 | 1-10 |
| PX | CN 205050303 U (GUANGDONG APPSCOMM CO., LTD.), 24 February 2016 (24.02.2016), description, paragraphs 0024-0064, and figures 1-4 | 1-10 |
| Y | CN 103810817 A (GUANGDONG APPSCOMM CO., LTD.), 21 May 2014 (21.05.2014), description, paragraphs 0056-0086, and figures 2-3 | 1-10 |
| Y | CN 104490397 A (XIN, Qin), 08 April 2015 (08.04.2015), description, paragraphs 0034-0040, and figures 1-2 | 1-10 |
| Y | US 2015269824 A1 (ZHANG, J.K.), 24 September 2015 (24.09.2015), description, paragraphs 0064-0066 | 2, 7 |
| Y | CN 102048521 A (THE HONG KONG POLYTECHNIC UNIVERSITY), 11 May 2011 (11.05.2011), description, paragraphs 0026-0046, and figures 1-3 | 1-10 |
| A | CN 204274428 U (SHENZHEN E-TECHCO SCIENCE AND TECHNOLOGY CO., LTD. et al.), 22 April 2015 (22.04.2015), the whole document | 1-10 |

☒ Further documents are listed in the continuation of Box C. ☒ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 17 August 2016 (17.08.2016) | **26 August 2016 (26.08.2016)** |

| Name and mailing address of the ISA/CN: State Intellectual Property Office of the P. R. China No. 6, Xitucheng Road, Jimenqiao Haidian District, Beijing 100088, China Facsimile No.: (86-10) 62019451 | Authorized officer **ZHANG, Xiao** Telephone No.: (86-10) **62085812** |

Form PCT/ISA/210 (second sheet) (July 2009)

## INTERNATIONAL SEARCH REPORT

International application No.

**PCT/CN2016/084730**

| C (Continuation). DOCUMENTS CONSIDERED TO BE RELEVANT | | |
|---|---|---|
| Category* | Citation of document, with indication, where appropriate, of the relevant passages | Relevant to claim No. |
| A | CN 103503041 A (KONINKL PHILIPS ELECTRONICS NV), 08 January 2014 (08.01.2014), the whole document | 1-10 |

Form PCT/ISA/210 (continuation of second sheet) (July 2009)

# INTERNATIONAL SEARCH REPORT
### Information on patent family members

International application No.

**PCT/CN2016/084730**

| Patent Documents referred in the Report | Publication Date | Patent Family | Publication Date |
|---|---|---|---|
| CN 105118236 A | 02 December 2015 | None | |
| CN 205050303 U | 24 February 2016 | None | |
| CN 103810817 A | 21 May 2014 | CN 103810817 B | 20 January 2016 |
| CN 104490397 A | 08 April 2015 | None | |
| US 2015269824 A1 | 24 September 2015 | US 2016163174 A1 | 09 June 2016 |
| | | US 9293023 B2 | 22 March 2016 |
| CN 102048521 A | 11 May 2011 | CN 102048521 B | 17 July 2013 |
| CN 204274428 U | 22 April 2015 | None | |
| CN 103503041 A | 08 January 2014 | JP 2014518666 A | 07 August 2014 |
| | | EP 2702575 A1 | 05 March 2014 |
| | | JP 2014518668 A | 07 August 2014 |
| | | EP 2702576 A1 | 05 March 2014 |
| | | CN 103503041 B | 18 May 2016 |
| | | WO 2012146957 A1 | 01 November 2012 |
| | | RU 2013153099 A | 10 June 2015 |
| | | CN 103493113 A | 01 January 2014 |
| | | US 2014191863 A1 | 10 July 2014 |
| | | US 2014142460 A1 | 22 May 2014 |
| | | WO 2012147036 A1 | 01 November 2012 |

Form PCT/ISA/210 (patent family annex) (July 2009)